(19) Europäisches Patentamt
European Patent Office
Office européen des brevets

(11) Publication number: 0 077 158 A1

(12) EUROPEAN PATENT APPLICATION

(21) Application number: 82305249.3

(22) Date of filing: 04.10.82

(51) Int. Cl.³: A 61 K 39/35, C 07 G 17/00

(30) Priority: 09.10.81 GB 8130625
13.10.81 GB 8130910

(43) Date of publication of application: 20.04.83
Bulletin 83/16

(84) Designated Contracting States: BE CH DE FR GB IT LI NL SE

(71) Applicant: BEECHAM GROUP PLC, Beecham House Great West Road, Brentford Middlesex (GB)

(72) Inventor: Whittall, Neil, 1 Lesbourne Court Lesbourne Road, Reigate Surrey (GB)

(74) Representative: Russell, Brian John et al, European Patent Attorney Beecham Pharmaceuticals Great Burgh Yew Tree Bottom Road, Epsom Surrey, KT18 5XQ (GB)

(54) **Modified allergens.**

(57) A process for preparing a polysarcosine modified allergen comprises binding polysarcosine in the form of a compound of formula (I):

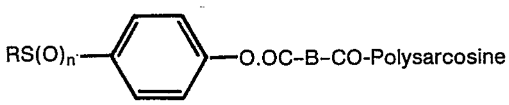

(I)

wherein R is a $C_{1-6}$ alkyl group, preferably methyl, n is 1 or 2, B is a hydrocarbon chain of 1 to 4 carbon atoms, optionally containing a double bond, and the polysarcosine is bound through its N-terminus; to an allergen. The modified allergen thus produced is useful in suppressing the production of IgE antibodies specific to unmodified allergen.

EP 0 077 158 A1

# MODIFIED ALLERGENS

This invention relates to a process for preparing modified allergens, to modified allergens that may be prepared by that process, and to the use of the modified allergens in the therapy of allergic humans.

Many people are allergic to allergenic materials such as pollens, weeds and house dust. Such allergies have been conventionally treated by the administration to the sufferer of repeated gradually increasing doses of the relevant allergen, to build up resistance to the allergen. This is known as desensitisation.

In our U K Patent No 1 282 163 is described one improved form of therapy, in which the allergenicity of a given allergen is reduced by treatment with glutaraldehyde. It is found that such material maintains its ability to stimulate the desired blocking antibody, and thus may be used in desensitisation therapy with a reduced risk of side effects.

An alternative approach to the problem is the modification of the allergen such that on administration it suppresses the production of IgE antibodies specific to the unmodified allergen. Such an approach

is described in Dutch Patent Application No. 7 709 025 (now also published as U.K. Patent No. 1 578 348), wherein it is stated that allergen-polyethylene glycol conjugates are capable of eliciting the therapeutically desirable effect of suppressing in particular allergen specific IgE production. These materials are also stated to be substantially non-allergenic and non-immunogenic.

It should be noted that although in this Dutch Patent Application it is suggested that other polymers such as polyvinylalcohols, polyvinylpyrrolidones, polyacrylamides and homopolymers of amino acids may be used in place of polyethylene glycol, only polyethylene glycols are illustrated.

In copending published European Patent Application No: 0038154 it is reported that allergens modified with polysarcosine have the ability to suppress the production of IgE antibodies specific to unmodified allergen.

A process for preparing certain polysarcosine modified allergens has now been discovered which differs from the processes described in the said European Patent Application.

Accordingly the present invention provides a process for preparing a polysarcosine modified allergen, which process comprises binding the polysarcosine to the allergen, characterised in that the polysarcosine is reacted in the form of a compound of formula (I):

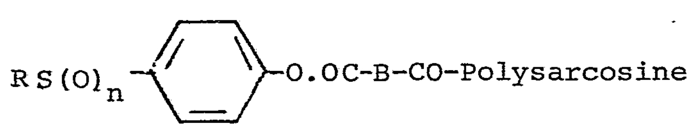

(I)

wherein R is a $C_{1-6}$ alkyl group, preferably methyl, n is 1 or 2, B is a hydrocarbon chain of 1 to 4 carbon atoms, optionally containing a double bond, and the polysarcosine is bound through its N-terminus.

When n is 1, the $RS(O)_n$ - moiety becomes RSO-. When n is 2, the $RS(O)_n$- moiety becomes $RSO_2$-.

This reaction may be carried out in conventional manner known for binding molecules to allergens, which methods are based on reacting active groups on the molecules with active groups on the allergens, and if necessary or convenient providing such active groups as a first step.

The allergen is preferably in the form of an extract of whole allergen, as is conventional. Suitable whole allergens from which the extract can be obtained include pollens, such as grass pollens, for example rye; weeds, such as ragweed; house dust mites; venoms, such as bee venom. Often the whole allergen will be ragweed, or a mixture of grasses, preferably a mixture of grasses.

Examples of B include $-CH_2-$, $-CH_2-CH_2-$, $-C(CH_3)=C(CH_3)-$ and -CH=CH-. Preferably B is $-CH_2-CH_2-$.

The polysarcosine for use in the process of this invention may have any convenient molecular weight. However, we have found that polysarcosine of molecular weight in the range 2000 to 12000 is suitable, more suitably 2000 to 9000. Polymers of molecular weight in this range are commercially available, but of course can be synthesised in conventional manner if desired or necessary. When used herein in relation to polysarcosine, molecular weights are number average molecular weights.

It will be appreciated by the skilled man that each allergen molecule will have a number of suitable sites for binding of polysarcosine molecules. It will be a routine matter for the skilled man, now we have discovered the advantages obtained thereby, to determine by simple experiment suitable binding levels for different allergens and different molecular weight polysarcosines to give the desired activity (IgE suppression). The degree of allergen substitution in the reaction can generally be controlled by addition of different amounts of activated polysarcosine.

The compounds of formula (I) are themselves novel, and therefore form a further aspect of the present invention.

The compounds of formula (I) may be prepared by the oxidation of a compound of formula (II):

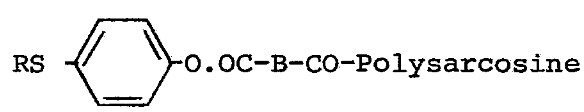

(II)

wherein R and B are as defined in formula (I).

This oxidation may suitably be carried out using excess hydrogen peroxide in glacial acetic acid. Other mild oxidising agents may also be used, for example, 3-chloroperoxy benzoic acid.

Depending on reaction conditions, a mixture containing variable proportions of compounds of formula (I) wherein n is 1 and n is 2 can be produced. By restricting the amount of oxidising agent used, for example, to one equivalent, the compound of formula (I) wherein n is 1 will predominantly be produced.

The compounds of formula (II) may be prepared by coupling polysarcosine to a compound of formula (III):

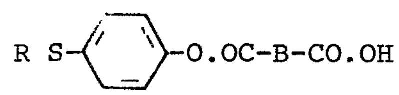

(III)

wherein R and B are as defined in formula (I).

This reaction may suitably be carried out by any of the conventional methods known in peptide chemistry, which usually involve activation of the acid function. In our hands one convenient procedure is to use hydroxysuccinimide and dicyclohexylcarbodiimide.

Also of course, other activated esters may be prepared using the appropriate percursor, for example - 4-nitrophenol or pentafluorophenol, in conjunction with a condensing agent such as a carbodiimide, particularly, dicyclohexylcarbodiimide.

The compounds of formula (III) may be prepared by coupling an acid HOOC-B-COOH to a compound of formula (IV):

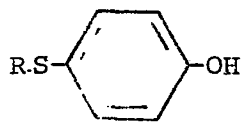

(IV)

wherein R is as defined in formula (I).

This reaction again can be carried out in conventional manner, which can conveniently involve using the acid in the form of an anhydride, such as succinic anhydride.

When using a dicarboxylic acid of formula HOOC-B-COOH in the preparation of compounds of formula (III) selective reaction of one only of the acid groups will be achieved using one equivalent of the compound of formula (IV) together with a suitable condensing agent, for example, dicyclohexylcarbodiimide in the presence of a catalytic amount of 4,4-dimethylaminopyridine.

The process of this invention is believed to have useful advantages.

For example, it is believed that a minimum of polysarcosine may be used in the reaction due to the high coupling efficiency leading to a low level of polysarcosine loss.

Also, it is believed that this process can yield materials having high levels of polysarcosine binding to the allergens.

Further, it is believed that this process can yield modified materials having a low level of retained allergenicity.

The polysarcosine modified allergens when prepared by the process of this invention are novel and form an important aspect of this invention. In such materials preferably the allergen is mixed grass, B is $-CH_2CH_2-$ and usefully the polysarcosine has a molecular weight of about 2000.

The modified allergens of this invention suppress IgE production specific to the unmodified allergen. They may therefore be used in the therapy of allergy in humans. For example, if a patient is allergic to ragweed, then a modified allergen according to this invention in which the allergen is ragweed would be used.

The modified allergens of the present invention may be employed as the active agents in compositions such as vaccines. Such compositions are well known to those skilled in the art and comprise a sterile liquid vehicle in which the active agent is dissolved or suspended. If suspended, the particles of active agent should be small enough not to block the orifice of an injection needle. Certain additives such as tyrosine are often included in such compositions and are believed to provide a support and prolonged slow release of ative material *in vivo*.

Usually a patient receiving treatment with such a composition is administered a number of injections, spread over a period of weeks or days.

It is also believed that the modified allergens of the invention may be active *via* other routes of administration, such as the nasal mucosa, when administration can be as a liquid spray or as a dry powder.

A preferred composition of this invention is as a vaccine.

The composition of this invention may, by way of illustration, suitably contain 1 to 10000 P.N.U. of modified material.

Normally, of course, doses towards the lower end of this scale will be more suitable for early in the therapy; doses towards the higher end of this scale for later in the therapy.

The following Examples illustrate the invention.

All temperatures are given in $^{o}C$.

Example 1

Preparation of 4-(methylmercapto)phenyl succinimidyl succinate

(a) 4-(Methylmercapto)phenyl hydrogen succinate

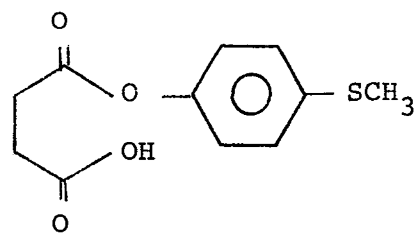

To 4-(methylmercapto)phenol (10 g, 0.071 mole) in chloroform (50 ml) were added triethylamine (14.4 g, 0.142 mole) and succinic anhydride (12.85 g, 0.128 mole). The mixture was stirred at ambient temperatures for 20 hours, the solution was washed with 0.1M hydrochloric acid (3 x 25 ml), dried over $MgSO_4$ and concentrated. Recrystallisation of the residue from chloroform at -20°C gave a white crystalline product 8.5 g, 58% of theoretical. Mpt 113°.

'H-nmr, $CDCl_3$ + TMS, δ: 2.47 (s, 3H, $SCH_3$);
2.83 (br.s, 4H, 2 $CH_2$);
7.15 (m, 4H, Ph).

Microanalysis: $C_{11}H_{12}O_4S$ requires: C, 54.99; H, 5.03; S, 13.34. Found: C, 54.97; H, 4.76; S, 13.20.

(b) 4-(Methylmercapto)phenyl succinimidyl succinate

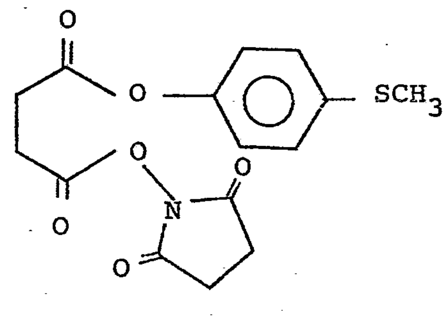

4-(Methylmercapto)phenyl hydrogen succinate (4 g, 0.017 mole) and N-hydroxysuccinimide (2.2 g, 0.019 mole) were dissolved in chloroform (50 ml), the solution cooled in ice-water and dicyclohexylcarbodiimide (4.12 g, 0.0 2 mole) in chloroform (20 ml) added. After 15 minutes at 4$^{o}$ the reaction was allowed to warm to room temperature and stirred for 5 hours. It was then filtered and the filtrate washed successively with 5% aqueous citric acid (20 ml), 5% aqueous sodium bicarbonate (20 ml) and water (20 ml). After drying over $MgSO_4$ evaporation gave a solid which was recrystallised from ethyl acetate/diethyl ether to give the product 5.4 g, 96% of theoretical. Mpt 98$^{o}$.

'H-nmr, $CDCl_3$ + TMS, δ: 2.47 (s, 3H, $SCH_3$);
2.81 (s, 4H, OSu);
3.0 (m, 4H, 2 $CH_2$);
7.10 (m, 4H, Ph).

Microanalysis: $C_{15}H_{15}NO_6S$ requires: C, 53.40; H, 4.48; N, 4.15; S, 9.50. Found: C, 53.23; H, 4.41; N, 4.22; S, 9.46.

Example 2

Preparation of activated polysarcosine

(a) N-(4-(Methylmercapto)phenyloxysuccinyl)-polysarcosine

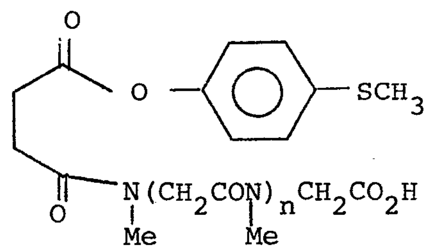

4-(Methylmercapto)phenyl succinimidyl succinate (0.08 g, 0.25 mmole) was stirred with polysarcosine, $\bar{M}_n$ = 2 100, (0.17 g, 0.081 mmole) and triethylamine (75µl, 0.54 mmole) in distilled and dried (3Å molecular sieves) N,N-dimethylformamide (4 ml) at 50-60° for 6 hours, then at ambient temperature for 20 hours. The solvent was evaporated in vacuo and the residue dissolved in water (5 ml). This solution was washed several times with ethyl acetate (5 x 3 ml) then lyophilised. The residue, 0.142 g, 76% of theoretical material was analysed by UV-spectrometry. Quantification of the absorbance of 285 nm, due to the methylmercaptophenyl moiety, indicated that complete modification of the N-terminal groups of the polysarcosine had been achieved.

(b) N-(4-(Methylsulphonyl)phenyloxysuccinyl)-polysarcosine

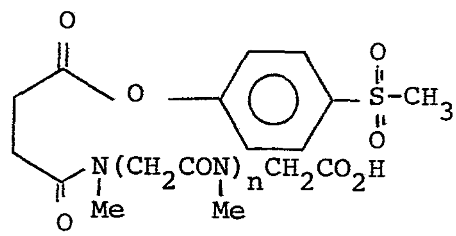

N-(4-(Methylmercapto)phenyloxysuccinyl)polysarcosine (0.13 g, 0.056 mmole) was dissolved in glacial acetic acid (2.6 ml) and stirred with hydrogen peroxide (30% w/v) (26 µl) at ambient temperature for 20 hours. Excess saturated sodium metabisulphite solution was slowly added to the cooled reaction mixture until this gave a negative starch/iodide test, and the solution was evaporated to dryness. The residue was dissolved in water (2 ml) and lyophilised to give a white solid (0.12 g, 91% of theoretical). Analysis by UV-spectrometry revealed the total lack of absorption at 255 nm, indicating complete oxidation of the methylmercapto group.

Example 3

Preparation of polysarcosine modified mixed grass pollen extract

Mixed grass pollen extract (400 mg) was dissolved in 0.1 M sodium borate, pH 9.0, at a concentration of 10 mg/ml. To this solution were added, at intervals over five hours, three portions of N-(4-(methylsulphonyl)-phenyloxysuccinyl)polysarcosine (as prepared in Example 2 but using polysarcosine of $M_n$=2750) (500 mg each). The pH was maintained at 9.0 by the addition of 1M sodium hydroxide as necessary. After the last addition the reaction mixture was incubated at 30°C for 18 hours.

The reaction mixture was filtered over an ultrafiltration membrane with a nominal molecular weight cutoff of 10,000 Daltons at 30 psi of nitrogen using 50 mM ammonium bicarbonate as eluant. The solution was allowed to concentrate to about 20 ml then diluted to about 200 ml and filtration continued. This cycle was repeated until the absorbances of the eluate at 236 and 280 nm had both fallen to a steady value below 0.2 absorbance units. The retentate was then removed from the ultrafiltration cell and lyophilised.

The product (560 mg) was characterised by amino acid analysis, primary amino group assay using 2,4,6-trinitro-benzenesulphonic acid, biuret assay, and a radioallergosorbent (RAST) inhibition assay, as shown in Table 2.

Example 4

Preparation of polysarcosine modified rye grass pollen extract

Rye grass pollen extract (80 mg) was dissolved in 0.1M sodium borate at pH 9.0, at a concentration of 10 mg/ml. To this solution was added, in one portion, N-(4-(methylsulphonyl)phenyloxysuccinyl)polysarcosine (as used in Example 3) (300 mg). The pH was maintained at 9.0 by the addition of 1M sodium hydroxide when necessary. The reaction mixture was incubated at 30°C for 17 hours.

The reaction mixture was filtered over an ultrafiltration membrane with a nominal molecular weight cutoff of 10,000 Daltons at 30 psi of nitrogen, using 50 mM ammonium bicarbonate as eluant. The solution was allowed to concentrate to about 2 ml then diluted to about 10 ml and filtration continued. This cycle was repeated until the absorbances of the eluate at 236 and 280 nm were both at a steady value below 0.2 absorbance units. The retentate was then removed from the ultrafiltration cell and lyophilised.

The product (100 mg) was characterised by amino acid analysis, primary amino group content, biuret assay, and a radioallergosorbent (RAST) inhibition assay, as shown in Table 2).

The method and results of a biological assay of this material are given below.

## Biological Results

### Suppression of the developing IgE response in BD 1 mice

### Method

Groups of 6 BD 1 mice were immunised intraperitoneally with 10 μg of rye grass pollen extract adsorbed onto 0.25 - 1.0 mg of aluminium hydroxide gel. On days 3, 5 and 7 the animals were injected intravenously with various amounts of the modified antigen (50 and 10 μg) in Bacto-haemmaglutination buffer (Difco*) (PBS) (0.5 ml) or diluent alone.

Serum was taken at 17 and 38 days and that from each group bulked at the time of bleeding. The antigen-specific IgE antibody in each bulked serum sample was titrated by a standard passive cutaneous anaphylaxis (PCA) test in two rats using a latent period of 48 hours. Results are expressed at $-\log_2$ from ¼ of the last dilution giving a positive result. A figure of 0 indicates a level of specific IgE below the limit of detection.

Table 1

| Group No. | Treatment Day 3, 5, 7 | PCA sp. IgE $-\log_2$ from ¼ Day 17 | 38 |
|---|---|---|---|
| 1 | Product from Example 4 (50 μg) | 0 | 0 |
| 2 | Product from Example 4 (10 μg) | 0 | 0.5 |
| 3 | PBS | 4 | 6 |

Table 2

| Example No. | % extract in conjugate (biuret) | Primary amino group content (μequiv/mg) | | % modification of extract in conjugate* | RAST inhibition n-fold reduction° |
|---|---|---|---|---|---|
| | | Calc. max.+ | Found | | |
| 3 | 59 | 0.27 | 0.17 | 37 | 20 |
| 4 | 59 | 0.29 | 0.22 | 24 | 48 |

+ Calculated from (fraction of extract in conjugate) x (primary amino group content of buffer control).

* Calculated from primary amino group figures.

o n-fold reduction relative to native extract.

0077158

CLAIMS

1. A compound of formula (I):

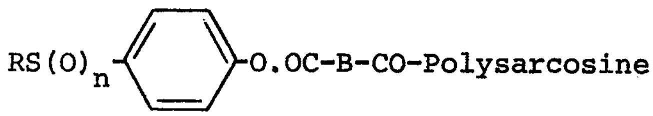 (I)

wherein R is a $C_{1-6}$ alkyl group, n is 1 or 2, B is a hydrocarbon chain of from 1 to 4 carbon atoms optionally containing a double bond, and the polysarcosine is bound through its N-terminus.

2. A compound according to claim 1, wherein R is a methyl group.

3. A compound according to claim 1 or claim 2, wherein n is 2.

4. A compound according to any one of claims 1 to 3, wherein B is $-CH_2-CH_2-$.

5. A compound according to any one of claims 1 to 4, wherein the polysarcosine has a molecular weight of from 2000 to 9000.

6. A process for preparing a compound according to any one of claims 1 to 5, which comprises oxidising a compound of formula (II):

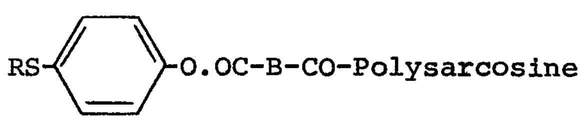 (II)

wherein R and B are as defined in formula (I).

7. A process for preparing a polysarcosine modified allergen, which comprises binding polysarcosine to the allergen, characterised in that the polysarcosine is reacted with the allergen in the form of a compound according to any one of claims 1 to 5.

8. Polysarcosine modified allergen when produced by a process according to claim 7.

9. A pharmaceutical composition comprising a polysarcosine modified allergen according to claim 8, together with a pharmaceutically acceptable carrier.

10. A composition according to claim 9 in the form of a vaccine.

European Patent Office

# EUROPEAN SEARCH REPORT

0077158

Application number

EP 82 30 5249

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,P X | EP-A-0 038 154 (BEECHAM) * the whole document * | 1,5,7, 9 | A 61 K 39/35 C 07 G 17/00 |
| P | EP-A-0 038 153 (BEECHAM) * the whole document * | 1,5,7, 9 | |
| A | US-A-4 256 732 (MALLEY) * title page; columns 1-4,7,8; claims 1-3 * | 1,9 | |
| A | US-A-4 191 668 (KATZ) * title page; columns 1-8,11,12; claims 1-4* | 1,5,9 | |
| A | US-A-4 180 562 (PATTERSON) * title page; columns 1-4 * | 1,9 | TECHNICAL FIELDS SEARCHED (Int. Cl. 3) |
| D,A | US-A-4 261 973 (W.Y. LEE) * title page; columns 1-4,17,18; claims 1-3 * | 1,5,9 | A 61 K 39/00 C 07 G 17/00 |

The present search report has been drawn up for all claims

| Place of search | Date of completion of the search | Examiner |
|---|---|---|
| THE HAGUE | 06-01-1983 | RAJIC M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document